# EUROPEAN PATENT APPLICATION

(11) **EP 4 549 429 A1**
(43) Date of publication of application: **07.05.2025**
(21) Application number: 23751316.3
(22) Date of filing: 29.06.2023
(51) Int. Cl.: C07C 407/00, C07C 409/24, C01B 15/026, C12P 7/40, A61L 2/18

(54) **METHOD AND SYSTEM FOR THE PRODUCTION OF DISINFECTION AND/OR STERILISATION SOLUTIONS**

(30) Priority: 30.06.2022 ES 202230591
(71) Applicant: Universitat Jaume I, 12071 Castelló de La Plana (Castellón) (ES); Estévez Company, Carlos, 08197 Sant Cugat del Vallés (Barcelona) (ES)
(72) Inventor: GARCIA-VERDUGO CEPEDA, Eduardo, 12006 Castelló de la Plana (ES); MACIÁ DELGADO, María, 12006 Castelló de la Plana (ES); LUIS LAFUENTE, Santiago Vicente, 12006 Castelló de la Plana (ES); SANS SANGORRÍN, Víctor, 12006 Castelló de la Plana (ES); SÁNCHEZ VELANDIA, Julián Eduardo, 12006 Castelló de la Plana (ES); ESTEVE COMPANY, Carlos, 08197 Sant Cugat del Vallés (Barcelona) (ES)
(74) Representative: Pons
(86) International application number: PCT/ES2023/070420
(87) International publication number: WO 2024/003436

(57) **Abstract**

The present invention relates to a method for obtaining disinfection and/or sterilisation solutions from a mixture of an alcohol and a dialkyl carbonate. Said mixture is placed in contact with O₂ to obtain hydrogen peroxide (H₂O₂) by means of a photocatalytic reaction, followed by treatment of the resulting mixture with a catalyst to obtain peroxycarboxylic acid and final dilution with water. The present invention also relates to a system for carrying out the process continuously, as well as the preparation on demand of the active disinfection and/or sterilisation solution obtained using same.

## Description

The present invention relates to a method and system for the production of disinfection and/or sterilisation solutions (hereinafter, active solutions) from simple, commercially available, cheap and non-toxic reagents.

Therefore, this invention can be framed within the field of disinfection/sterilisation products and their production.

### BACKGROUND OF THE INVENTION

Due to their high potency at low concentrations and temperatures, liquid formulations based on peroxycarboxylic acids (e.g. peracetic acid) are widely used in low-temperature sterilisation processes of reusable and immiscible medical devices (e.g. disinfection of medical equipment, low temperature sterilisation of dentures, plastic implants, syringes, thermally sensitive nutrient media, disinfection of haemodialysis systems and decontamination of liquid and solid medical waste in hospitals). Some of these compositions have been disclosed in the following documents: US8263151, US20090314652, US5200189, and in: Laura Dominguez Henao et al. Chemosphere 213 (2018) 25-40.

The reason for the excellent and rapid antimicrobial effects of peroxycarboxylic acids is their specific ability to penetrate through the cell membrane. In the cell, the peroxyacid irreversibly alters the enzyme system, which leads to the destruction of the microorganism. Currently, products based on peroxyacids (e.g., peracetic acid) are used as highly effective biocides in a wide range of applications.

The main use of peroxycarboxylic acids, especially peracetic acid (PAA), is as a bactericide and fungicide agents. Thus, for example, regulations allow the use of peracetic acid as a disinfectant in the washing and rinsing water for raw and processed fruits and vegetables, meat and eggs (direct contact with food) and as a sanitiser on surfaces in contact with food. Similarly, PAA can be used in a wide temperature spectrum (0 to 40°C), in *cleaning-in-place* processes and in environments saturated with carbon dioxide. It can also be used with hard water. Furthermore, protein residues do not affect its efficiency. So far, no microbial resistance to PAA has been reported, being effective in a wide spectrum of pH, from values of 3.0 to 7.5.

PAA formulations have also been registered for use as a sanitiser, disinfectant, and sterilant by the European Chemicals Agency (ECHA) and the US Environmental Protection Agency, and as an antimicrobial agent by the US Food and Drug Administration (FDA) and the US Department of Agriculture.

PAA that is used as sanitiser is obtained by combining aqueous mixtures of two substances: acetic acid and hydrogen peroxide. PAA is conventionally prepared by reacting concentrated acetic acid (AA) and concentrated hydrogen peroxide in the presence of strong, homogeneous acid catalysts (e.g., 1-20% v/v sulphuric acid) to catalyse the reaction toward chemical equilibrium. The use of concentrated solutions of hydrogen peroxide has the drawback of the high potential for explosiveness both in transport and during manufacture in a chemical plant. In this mixture, the added reagents (e.g., acids, oxidising agents, stabilisers, etc.) must contain excess quantities to prevent breakdown during transport, which is another major drawback. Different concentrations of PAA are required depending on the final application. PAA concentrations up to 15% are normally used for water treatment, sanitising, disinfecting and sterilising in the food and beverage industry, in laundries and for medical applications. Higher concentrations of PAA, up to 40%, are used exclusively for oxidation reactions. To minimise the impact of transportation costs, PAA mixtures are produced at relatively high concentrations and then diluted at the point of use. However, these mixtures are dangerous due to their corrosive, oxidising and explosive properties, and require costly safety measures for their production and transport. In addition, peroxycarboxylic acids such as PAA have well-known chemical drawbacks; namely, they are relatively unstable in solution and break down into the corresponding carboxylic acids and oxygen.

There are methods of making peroxycarboxylic acids using hydrogen peroxide and different compounds as acid precursors, such as lactide and glycolide (US2021238135A1) or the corresponding polycarboxylic acid esters (WO9828267A1, WO9534537A1).

Regarding the synthesis of hydrogen peroxide, it can be obtained using anthraquinones as mediators in the presence of hydrogen, oxygen and a palladium catalyst (Jose M. Campos-Martin, et al. Angew. Chem. Int. Ed. 2006, 45 , 6962-6984).

On the other hand, different methods have been reported to manufacture peroxycarboxylic acid solutions *in situ* and at the point of use. For example, PAA can be generated by dissolving an activator (tetra-acetyl ethylenediamine) and a salt (sodium perborate or sodium percarbonate) in water, or *in situ* adding sodium hydroxide to a mixture of triacetin and hydrogen peroxide (US8546449). Different flow systems have also been reported for the generation of peroxides or peroxycarboxylic acids (WO2019191387A1, WO2008047263A2, WO0110215A1). However, these systems have technical, environmental and economic problems, such as expensive materials, the use of toxic and hazardous reagents, short life of the catalysts, and low concentration of PAA yield, among other drawbacks, which limit the use of these systems.

In view of the foregoing, the present invention proposes a method and system that resolve the limitations present in the methods known in the state of the art, providing active solutions on demand and generated *in situ* from simple, commercially available, cheap and non-toxic reagents.

### DESCRIPTION OF THE INVENTION

The present invention relates to a method and system for the continuous production, on demand and at the point of use, of active disinfection/sterilisation solutions comprising a peroxycarboxylic acid from simple, commercially available, cheap and non-toxic reagents which can act in disinfection and/or sterilisation processes. The method only requires basic and common equipment or system in continuous mode, and therefore, economic and easy to implement and use at the point of use ("*in situ*").

A first aspect of the invention relates to a method for the preparation of an active disinfection and/or sterilisation solution comprising the following stages:
a) a liquid mixture of a primary or secondary alcohol and a dialkyl carbonate is contacted with O₂ in the presence of a photocatalyst and UV light to obtain hydrogen peroxide (H₂O₂) by reaction of O₂ with the alcohol present in the mixture through a photocatalytic reaction,
b) the solution obtained in stage a) is placed in contact with a catalyst of the perhydrolysis reaction of the dialkyl carbonate, same being either non-enzymatic or enzymatic, at a temperature between 25°C and 80°C, to form peroxycarboxylic acid by reaction of the hydrogen peroxide formed in the previous stage a) and the dialkyl carbonate in the original mixture,
c) the solution obtained in stage b) is diluted with water to a concentration of said solution in water equal to or less than 10% v/v.

The diluted solution in stage c) will therefore have a maximum of 10 mL of the solution obtained in b) for every 100 mL of total diluted solution.

In a preferred embodiment, the starting mixture in stage a) comprises between 20-80% v/v of alcohol and the rest of dialkyl carbonate. More preferably, between 40-60% v/v of the alcohol.

In a preferred embodiment, the primary or secondary alcohol has the following formula: R₁-CH(OH)-R₂, where R₁ is H or a C1-C6 alkyl group and R₂ is a C1-C6 alkyl.

In a preferred embodiment, the primary or secondary alcohol is selected from the list comprising: isopropanol, ethanol, methanol, butanol, and glycerol. More preferably, the alcohol is isopropanol or ethanol.

The term "dialkyl carbonate" includes carbonates bound to two alkyl groups (alkyl-O-(C=O)-O-alkyl) and cyclic carbonates in which an alkylene group is attached to the -O-(C=O)-O- group, forming a cycle.

In a preferred embodiment, the dialkyl carbonate has the following formula: R₃-O-(C=O)-O-R₃ where R₃ is a linear or branched C1-C6 alkyl, optionally substituted with at least one hydroxyl (-OH) or a C1-C4 alkoxy group or combinations thereof.

In another preferred embodiment, the dialkyl carbonate is cyclic and has the following formula: 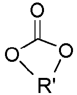 where R' is a C2-C6 alkylene group optionally substituted with at least one hydroxyl (-OH), a C1-C4 alkyl group, a C1-C4 alkoxy or combinations thereof.

In a preferred embodiment, the dialkyl carbonate is selected from the list comprising: dimethyl carbonate, diethyl carbonate, propylene carbonate and glycerol carbonate and, more preferably, the dialkyl carbonate is dimethyl carbonate, diethyl carbonate or carbonate glycerol.

The use of dialkyl carbonates as peroxycarboxylic acid precursors is advantageous, as they are excellent solvents for a wide variety of organic and inorganic materials and, as such, are used in a variety of applications and compositions, including, but not limited to, cleaners, degreasers, dyes, fibres, plastics, batteries, as a gelling agent for clays, and as a curing agent/accelerator for foundry sand resins. They are economical, non-toxic, safe, non-corrosive and non-irritating, therefore suitable for use in applications involving human contact.

In stage a) of the method, hydrogen peroxide (H₂O₂) is generated by a photocatalytic process of hydrogen transfer from an H donor (alcohol) to dissolved O₂ through the help of an excited photocatalyst. This reaction is preferably carried out in a photocatalytic reactor. The photocatalyst can be immobilised in the photocatalytic reactor or else dissolved in the starting alcohol/dialkyl carbonate mixture.

In a preferred embodiment, the photocatalyst used in stage a) is selected from the list comprising: anthraquinone, 2-bromomethylanthraquinone, 1,8-dihydroxyanthraquinone, anthraquinone-2-carboxylic acid, anthraquinone 2-sulphonate sodium, 2-tert-butylanthraquinone, 2-methylanthraquinone, 2-ethylanthraquinone.

Preferably, the photocatalyst is used in a concentration between 5 and 10 mM.

In a preferred embodiment, the photocatalyst is immobilised in a polymeric resin, which may or may not contain ionic liquids. Preferably, the polymeric resin is a chloride functionalised divinylbenzene crosslinked polystyrene resin. More preferably, the polymeric resin is Merrifield resin (2% divinylbenzene crosslinked polystyrene and chloride functionalised ).

An "ionic liquid" is understood to be a chemical entity made up of ions, that is, salts that have a melting temperature below the boiling point of water (100 °C at sea level) and that are thermally and chemically stable. These liquid units can be immobilised on resin by chemical adsorption or by covalent bonding.

As for the light administered for the production of said photochemical reaction, it is in the UV range, as indicated above, more preferably, the light administered is of a wavelength of 365 nm. An LED lamp or any UV light lamp can be used for this.

Preferably, the reaction of stage a) is carried out at room temperature (approx. 20-25°C) and/or for a period between 10 minutes and 2 hours, more preferably, between 30 minutes and 1 hour.

In the second stage b) of the method, the reaction of the hydrogen peroxide generated in stage a) with the dialkyl carbonate takes place to give the corresponding peroxycarboxylic acid. This reaction takes place in the presence of either a non-enzymatic or enzymatic catalyst. In the case of a non-enzymatic catalyst, this may be a catalyst containing Lewis acid groups. Preferably, a heterogeneous catalyst containing acid groups (e.g., strongly acidic cation exchange resins of the acrylate type or sulphonated polystyrene such as amberlite, nafion, or MOFS, i.e., metal organic frameworks). In the enzymatic case, it can be a lipase enzyme, such as *Fusarium solani pisi* lipase, *Candida antarctica* lipase, *Geobacillus thermoleovorans* IHI-91 lipase, *Kurtzmanomyces spec.* lipase. Preferably, CALB lipase (*Candida antarctica* lipase B) immobilised on a polymer support.

Preferably, stage b) is carried out in a fixed bed reactor.

In a preferred embodiment, the amount of enzyme used, preferably CALB, is between 0.1 and 0.6 g/mL of the reactant mixture.

In a preferred embodiment, the reaction of stage b) is carried out at a temperature between 35 and 45°C, more preferably at 40°C.

In a preferred embodiment, the reaction of stage b) is carried out for a time of between 20 and 30 minutes.

In a preferred embodiment, the method is carried out continuously and/or at the point of use of the solution. By continuously supplying a flow of reagents, it is possible to carry out the method continuously without the need to carry out any separation and/or purification stage.

A second aspect of the invention relates to a system for carrying out the method disclosed in the first aspect of the invention. The system is configured to be able to carry out the method continuously.

The system comprises:
- a photocatalytic reactor equipped with a UV light source and an O₂ inlet configured to carry out the reaction of stage a),
- a liquid pump configured to introduce the alcohol/dialkyl carbonate starting mixture, contained in a vessel, into the photocatalytic reactor,
- a reactor connected in series with the photocatalytic reactor configured to receive the solution obtained in stage a) and carry out the reaction in stage b),
- a dilution system configured to add water to the solution resulting from the second reactor. This equipment preferably consists of a line that is attached to the outlet of the reactor where the water is pumped.

In a preferred embodiment, the photochemical reactor comprises a pressure regulator. In another preferred embodiment, the system comprises a compressor located at the O₂ inlet configured to introduce O₂ under pressure into the reactor.

In a preferred embodiment, the system comprises flow sensors, located at the inlet and outlet of each reactor, to control the supply of reagents to the system. Furthermore, it may comprise a light sensor connected to the UV light source in order to control the light applied to the photocatalytic reactor.

The system thus configured, in which the reactors are connected and the pumps and regulators can be automatic and programmable to control the continuous flow of reagents, allows obtaining the active solution on demand, *in situ,* without the need for any further separation and/or purification stage.

A final aspect of the invention relates to an active disinfection/sterilisation solution, preferably obtained by means of the method and/or system defined above, comprising: a peroxycarboxylic acid, hydrogen peroxide, at least one alcohol and water.

In a preferred embodiment, the solution comprises:
Water at a concentration of 90 to 99.9% v/v, and a mixture of at least three components (peroxycarboxylic acid, hydrogen peroxide and alcohol) the total sum of which being a concentration from 0.1 to 10% v/v.

The solution can also comprise the ketone that is formed in stage a) of the process. In this case, the solution would have between 0.1 and 10% v/v of the mixture formed by peroxycarboxylic acid, hydrogen peroxide, ketone and alcohol.

The peroxycarboxylic acid present in the active cleaning, disinfection and/or sterilisation solution is formed in the second stage of the method (stage b) by reacting hydrogen peroxide with a dialkyl carbonate, as described in the first aspect of the invention. Hydrogen peroxide and the alcohol (primary or secondary as described in the first aspect of the invention) are reagents that do not react to completion in the method of the invention and, therefore, a portion remains unreacted in the final mixture.

Also, the alcohol is formed in stage b) after the reaction of hydrogen peroxide with a dialkyl carbonate. The water present in the solution corresponds to the dilution water. The synergistic combination of all the components enhances the disinfection/sterilisation properties compared to any one of them independently.

In addition to peroxycarboxylic acid, hydrogen peroxide and alcohols also have bacteriostatic properties (Adrian Man, *Revista Român* 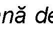 *de Medicin* 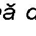 *de Laborator.* 25 (4), 2017, 335-34), being also an active ingredient of the final solution.

The active solution can be used to achieve a high level of disinfection and chemical sterilisation in different sectors such as for example, the health sector for the cleaning of medical and surgical devices. It can also be used for cleaning tools, surfaces, instruments, cleaning in place (CIP) pipes and other objects in general that are used in fields such as food and beverage handling, wastewater treatment plants or in any other field requiring a high level of disinfection treatment or chemical sterilisation.

The use of safe and low toxicity chemical compounds for the generation of hydrogen peroxide and peroxycarboxylic acids reduces or eliminates the hazard level of the system during the life cycle of the active solution.

In addition, it does not require the isolation of the active compounds (peroxycarboxylic acids and H₂O₂) nor their storage and transport at high concentrations with the additional risk that this entails, since the active solution can be generated on demand at the point of use. Thus, there would be no possibility of overpressure or explosions that can have catastrophic results. In addition to eliminating the need for transportation, the invention avoids long-term storage of the active solution, which would not only entail additional risks, but would also require the addition of various stabilising agents (e.g. stability for at least one year, as required for regulated chemical substances) with consequent increased costs.

### Definitions:

The term "primary alcohol" relates to an alcohol having the hydroxyl group attached to a primary carbon atom. It can also be defined as a molecule containing a "-CH₂OH" group.

The term "secondary alcohol" relates to an alcohol that has the hydroxyl group connected to a secondary carbon atom. It can also be defined as a molecule that contains a "-CHOH" group.

The term "alkyl" relates in the present invention to a radical formed by an aliphatic, linear or branched hydrocarbon chain, for example, methyl, ethyl, n-propyl, i-propyl, n-butyl, tert-butyl, sec-butyl, n-pentyl, etc. A C1-C6 alkyl is an alkyl having between 1 and 6 carbon atoms.

The term "alkoxy" relates to an -O-alkyl radical such as, for example, methoxy, ethoxy, or propoxy. A C1-C4 alkoxide relates to an -O-alkyl group where the alkyl group has between 1 and 4 carbon atoms.

The term "alkylene" relates to a divalent radical derived from an alkane such as - CH₂CH₂-, -CH₂CH₂CH₂- and -CH₂CH₂CH₂CH₂-. A C2-C6 alkylene is an alkylene comprising between 2 and 6 carbon atoms.

The term "active solution" is defined as a solution that provides the desired cleaning, disinfection and/or sterilisation effect, inactivating or destroying microorganisms. The active solution of chemical agents that shows the ability to inactivate or destroy microorganisms is described as a "biocidal" solution. In particular, the active solution of the present invention comprises the components listed above in the last aspect of the invention.

"Minimum biocidal concentration" relates to the lowest concentration of a biocidal agent that, for a specified contact time, will produce a lethal and irreversible reduction in the viable population of target microorganisms. The effectiveness can be measured by the logarithmic reduction in viable microorganisms after treatment.

The term "sanitation" relates to the physical removal of contaminating debris, dust, or material. It can be defined as the removal of contamination from an article to the extent required for further processing or intended use [ISO/TS 11139]. Sanitisation will reduce the number of microorganisms, as well as dirt, allowing better contact with the surface to be disinfected or sterilised and reducing the risk of dirt adhering to the surface. Removal of dirt will also reduce the risk of inactivation of a chemical disinfectant and the multiplication of microorganisms.

The term "disinfection" relates to the destruction or elimination of microorganisms to a level that is not harmful to health and safe to handle. This process does not necessarily include the destruction of bacterial spores. As used herein, the term "disinfectant" relates to an agent that kills all vegetative cells, including the most renowned pathogenic microorganisms, using the method described by A.O.A.C. (Association of Official Analytical Chemists) [Use Dilution Methods, Official Methods of Analysis of the Association of Official Analytical Chemists, paragraph 955.14 and applicable sections, 15th Edition, 1990 (EPA Guideline 91-2)]. As used herein, the term "high-level disinfection" or "high-level disinfectant" relates to a compound or composition that kills substantially all organisms, except high levels of bacterial spores, and is performed with a chemical germicide approved for sale as a sterilant by the FDA. As used herein, the term "intermediate level disinfection" or "intermediate level disinfectant" relates to a compound that kills mycobacteria, most viruses, and bacteria with a chemical germicide registered as a tuberculocidal by the EPA (Environmental Protection Agency). As used in this document, the term "low-level disinfection" or "low-level disinfectant" relates to a compound or composition that kills some viruses and bacteria with a chemical germicide registered as a hospital disinfectant by the EPA.

The term "sterilisation" relates to the complete destruction or elimination of microorganisms, including bacterial spores. "Sterile state" means to be free of viable microorganisms. A validated sterilisation process is used to obtain a product free of viable microorganisms.

The term "peroxycarboxylic acid" relates to a compound comprising the group -(CO)-O-OH. Therefore, the compounds comprising the group -O-(CO)-O-OH obtained in the present invention are considered to be peroxycarboxylic acids.

Throughout the description and claims the word "comprises" and its variants are not intended to exclude other technical characteristics, additives, components or stages. For those skilled in the art, other objects, advantages, and features of the invention will emerge in part from the description and in part from the practice of the invention. The following examples and figures are provided by way of illustration, and are not intended to be limiting of the present invention.

### BRIEF DESCRIPTION OF THE FIGURES

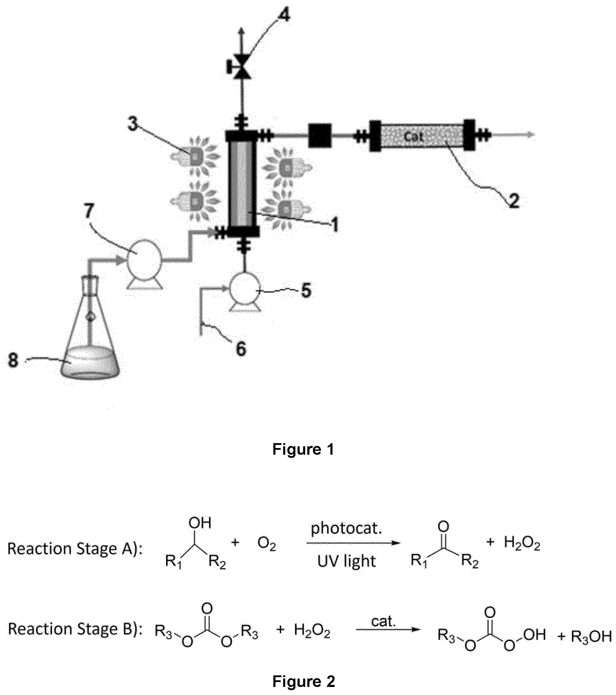

**Figure 1** shows a schematic representation of the system of the present invention where the numerical references have the following meaning: (1) Photocatalytic reactor, (2) fixed bed reactor, (3) UV light lamps, (4) pressure regulator, (5) compressor, (6) O₂ inlet, (7) liquid pump, (8) alcohol/dialkyl carbonate starting mixture container.
**Figure 2** shows a diagram of the reactions that occur in the photocatalytic reactor (reaction of stage a)) and in the second reactor (reaction of stage b)).

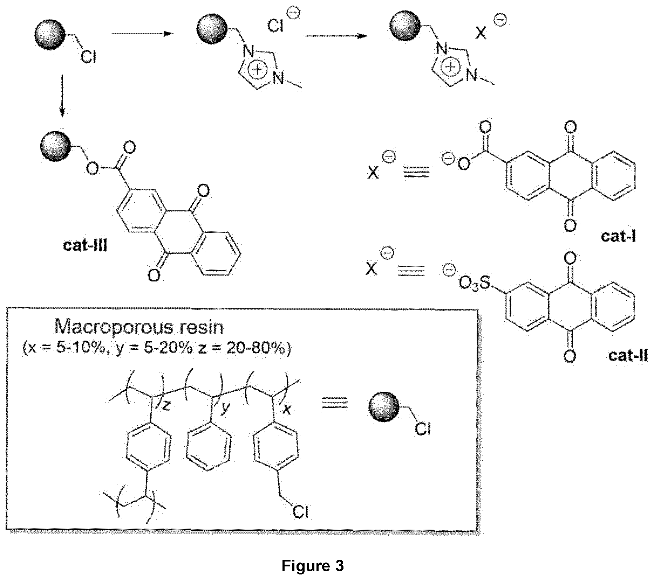

**Figure 3** shows a schematic representation of the synthetic route used to obtain the immobilised Cat-I, Cat-II and Cat-III photocatalysts obtained in Example 4.

### EXAMPLES

### Example 1. Continuous flow system for H₂O₂ on-demand synthesis.

This example demonstrates the production of H₂O₂ under the conditions that the process of the invention will be carried out continuously and that is shown in example 2.

0.5 g of the anthraquinone-2-carboxylic acid (photocatalyst) was dissolved in 250 mL of isopropanol (7.93 mM) in a 250 mL Erlenmeyer flask using magnetic stirring at room temperature. This solution was pumped into the flow system. The flow system consisted of a tube-in-tube reactor consisting of an internal ultrafiltration ceramic membrane (γ-Al₂O₃ membrane) and an external quartz tubing. The membrane outlet was connected to a back pressure regulator (BPR, 500 psi). The membrane was internally fed with air using an air pump. The air penetrated through the porous ceramic membrane and came into direct contact with the stream of alcohol solution simultaneously fed into the annular space. The lower and upper flanges were obtained by 3D printing and had inlet and outlet tubing located perpendicular to the flow direction of the liquid stream and tangentially to the quartz tube, in the horizontal plane and on top on opposite sides. UV lamps (9W, 365 nm) were used as the light source. The reactor volume was 20 mL and, when the system was filled, the output flow was 0.434 mL/min with a residence time of 46 minutes. After 3.5 hours of system stabilisation, samples of the output solution were collected at different time intervals using a fraction collector, with a collection time of 15 minutes in each fraction.

Table 1 summarises the results obtained with this flow system. The H₂O₂ concentration was determined by redox titration. The H₂O₂ concentration obtained was in the range of 3000-3500 ppm, which is in the H₂O₂ concentration range required for the synthesis of peracids for disinfection and sterilisation applications.

**Table 1. H₂O₂ synthesis using a tubular flow reactor.**

| | | | |
|---|---|---|---|
| 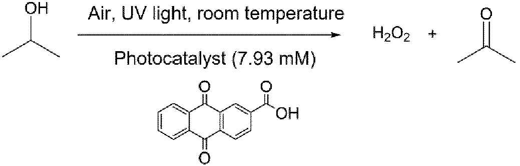 | | | |

| **Entry** | **Time (h)** | **[H₂O₂] (M)** | **[H₂O₂] (ppm)** |
|---|---|---|---|
| 1 | 3.75 - 4 | 0.1050 | 3570 |
| 2 | 4.75 - 5 | 0.1051 | 3573 |
| 3 | 5.75 - 6 | 0.1046 | 3556 |
| 4 | 6.75 - 7 | 0.0848 | 2883 |
| 5 | 7.75 - 8 | 0.1000 | 3400 |
| 6 | 8.25 - 8.30 | 0.1083 | 3682 |

### Example 2. Continuous flow system for the on-demand production of a peroxycarboxylic acid solution.

The first stage was the synthesis of H₂O₂ using the same system as shown in Example 1. In this example, the input stream feeding the first reactor was a 7.93 mM solution of photocatalyst in dimethyl carbonate (DMC) and isopropanol (IPA) in a 1:1 (v/v) ratio. The H₂O₂ concentration obtained was determined by redox titration, being 0.2846 M. The outlet current from the first stage was connected to a second reactor for the synthesis of peracids. This reactor consists of a cooled fixed bed reactor, packed with the supported enzyme. The active solution was pumped through the catalytic fixed bed, initially using a flow rate of 0.257 mL/min at 40 °C. Under these conditions, a residence time of 22.9 min was estimated, calculated taking into account the free volume of the reactor. Several samples of the output solution were collected at different time intervals using a fraction collector, with a collection time of 15 min for each fraction. Table 2 summarises the results obtained under these conditions. Peracid concentration was determined by HPLC analysis with MTS (p-tolyl methyl sulphide). The system was quite stable providing a mean value for the concentration of the corresponding DMC-derived peroxycarboxylic acid of 3100 ppm for at least four hours. After this time, the flow was increased to 1 mL/min to assess the productivity of the system. Although this increased flux reduced the residence time from 22.9 to 588 min, the concentration of the corresponding DMC-derived peroxycarboxylic acid remained at a similar level, averaging 2650 ppm.

**Table 2. Synthesis of the corresponding DMC-derived peroxycarboxylic acid using the continuous flow system.**

| **Entry** | **Flow (mL/min)** | **Time (min)** | **[peroxycarboxylic acid] (M)** | **[peroxycarboxylic acid] (ppm)** |
|---|---|---|---|---|
| 1 | 0.257 | 60 - 75 | 0,033 | 3038 |
| 2 | 0.257 | 90 - 105 | 0,030 | 2762 |
| 3 | 0.257 | 120 - 135 | 0.033 | 3038 |
| 4 | 0.257 | 150 - 165 | 0.034 | 3130 |
| 5 | 0.257 | 180 - 195 | 0.035 | 3222 |
| 6 | 0.257 | 210 - 225 | 0.036 | 3314 |
| 7 | 0.257 | 240 - 255 | 0.034 | 3130 |
| 8 | 1.000 | 285 - 300 | 0.029 | 2669 |
| 9 | 1.000 | 300 - 315 | 0.029 | 2669 |
| 10 | 1.000 | 360 - 375 | 0.027 | 2485 |
| 11 | 1.000 | 375 - 390 | 0.030 | 2762 |
| 12 | 1.000 | 420 - 435 | 0.029 | 2669 |

### Example 3. Validation of the bactericidal activity of the active solution.

For the validation of the active solution, firstly, an active solution was generated using the same flow system shown in Example 2, obtaining an H₂O₂ concentration of 0.2743 M and a peroxyacid concentration of 0.0265 M. Bactericidal activity assays were carried out using eight diluted aqueous solutions from the active solution prepared with the flow system. Table 3 shows the final concentration of peroxyacid and H₂O₂ obtained by dilution.

**Table 3. Range of concentrations of DMC/ H₂O₂-derived peroxycarboxylic acid [PCA] evaluated in the bactericidal efficacy tests for different active solutions.**

| Active Solution | Active solution concentration after dilution with water (% v/v) | [PCA] (M) | [H₂O₂] (M) |
|---|---|---|---|
| 1 | 80% | 0.0212 | 0.2194 |
| 2 | 40% | 0.0106 | 0.1097 |
| 3 | 20% | 0.0053 | 0.0549 |
| 4 | 8% | 0.0212 | 0.0219 |
| 5 | 4% | 0.00106 | 0.0110 |
| 6 | 0.8% | 0.000212 | 0.0022 |
| 7 | 0.4% | 0.000106 | 0.0011 |
| 8 | 0.2% | 0.000053 | 0.055 |

Three reference microorganisms (*Pseudomonas aeruginosa* CECT 116, *Staphylococcus aureus* CECT 239, and *Enterococcus hirae* CECT 4081) were exposed to the action of these solutions. The microorganisms were exposed to the solution at 20 °C for 60 minutes following the standard for "Chemical disinfectants and antiseptics - Quantitative suspension test for the evaluation of bactericidal activity in the medical area EN 13727:2012+A2:2015.a" [https:/ /www.une.org/encuentra-tunorma/busca-tu-norma/norma?c=N0055967]. The assay measured the decrease in the number of cells per unit volume due to the action of the sterilising agent. The results of the bactericidal test are shown in Table 4.

**Table 4. Results of the bactericidal efficacy test of the active solutions 6, 7 and 8 of Table 3 according to the EN standard 13727:2012+A2:2015.a**

| **Microorganis m** | **Initial number of microorg anisms per mL, Nᵢ** | **Number of surviving microorganisms per mL, N_{f},** | | | **PCA and H₂O₂ concentration threshold at which bactericidal activity is observed** |
|---|---|---|---|---|---|
| | | **Active solution 6 (0.8%), N_{f}** | **Active solution 7 (0.4%), N_{f}** | **Active solution 8 (0.2%), N_{f}** | |
| *P.aeruginosa* | 3.4 × 10⁷ | ≤1.4×10² | ≤1.4×10² | ≥3.3×10⁴ | [PCA]=0.001% (10 ppm) |
| | | LogR≥5.38 | LogR≥5.38 | LogR≤3.01 | |
| | | | | | [H₂O₂]= 0.01% (100 ppm) |
| *S. aureus* | 3.6 × 10⁷ | ≤1.4×10² | ≤1.4×10² | ≥3.3×10⁴ | [PCA]=0.001% (10 ppm) |
| | | LogR≥5.41 | LogR≥5.41 | LogR≤3.04 | |
| | | | | | [H₂O₂]=0.01% (100 ppm) |
| *E. hirae* | 3.8 × 10⁷ | ≤1.4×10² | ≤1.4×10² | ≥3.3×10⁴ | [PCA]=0,001% (10 ppm) |
| | | LogR≥5.43 | LogR≥5.43 | LogR≤3.06 | |
| | | | | | [H₂O₂]=0.01% (100 ppm) |
| ^{a}Log R is the logarithm of the number of microorganisms that have died. | | | | | |

According to the standard, the required reduction of the initial bioburden, measured in number of cells per mL in logarithmic units (Log R = LogNᵢ - LogN_{f}) where Nᵢ is the initial bioburden and N_{f} is the final cell concentration, should be greater than 5. This condition was met for active solutions 1 through 7. Solution 7 was the lowest concentration of active ingredients that satisfied the bactericidal condition in this test. The concentration of peroxyacid in solution 7 was 0.001% v/v and the concentration of H₂O₂ was 0.01% v/v. These concentrations were compared to those of US and EU approved high-level disinfectants/sterilisers in Table 5.

**Table 5. Comparison between concentrations of active ingredients in Active Solution 7 and EU-US approved liquid biocides.**

| **Biocide** | **Minimum biocidal concentration** | **Status** |
|---|---|---|
| Hydrogen peroxide / PCA (Active solution 7) | 0.01% / 0.001% | This study |
| Hydrogen peroxide / Peracetic acid | 1.0% / 0.08% | EU-US approved |
| Hydrogen peroxide / Peracetic acid | 7.5% / 0.23% | EU-US approved |
| Glutaraldehyde | ≥ 2% | EU-US approved |
| Orto-phtalaldehyde | 0.55% | EU-US approved |

It must be taken into account that the bactericidal concentration of the active ingredients of the active solution 7 was two orders of magnitude lower than the concentrations of the commercial disinfectant/sterilant liquids approved by the health authorities of the EU and the USA.

### Example 4. Immobilised photocatalytic systems for the production on demand of a H₂O₂ solution.

**Preparation of immobilised photocatalysts on supports containing ionic liquid units.** A 1 g suspension of high loading (5.5 meq Cl/g_{polymer}) Merrifield type macroporous polymer resin (Merck, CAS 55844-94-5: chloromethylpolystyrene with 5.5% divinylbenzene as crosslinking agent) in methyl imidazole was stirred by orbital shaking at 150 revolutions per minute at 80°C for 24 h. Subsequently, the material was washed with acetonitrile (25 mL x 3), dichloromethane (25 mL x 3) and methanol (25 mL x 3). Subsequently, the resin was dried under vacuum at 50°C. The resulting resin was suspended in 20 mL in a 25% ammonia solution where it was kept stirring at room temperature for 2h. The resulting resin was washed with methanol (25 mL x 3) and placed in contact with a solution of N,N-dimethylformamide containing 1.5772 g of anthraquinone-2-carboxylic acid for the preparation of immobilised photocatalyst cat-I or alternatively 2.0264 g of anthraquinone -2-sulphonic acid for the preparation of the immobilised photocatalyst cat-II. The corresponding suspensions were maintained under orbital stirring at room temperature (150 revolutions per minute) for 24 h. The resulting polymeric resin was filtered and washed with DMF (25 mL x 3), acetonitrile (25 mL x 3) and methanol (25 mL x 3). Finally, the catalysts were vacuum dried overnight at 50°C for further use in hydrogen peroxide photosynthesis.

**Preparation of immobilised photocatalysts on supports without ionic liquids.** The immobilised **cat-III** photocatalyst was prepared by suspending 0.3 g of the high-charge (5.5 meq Cl/g_{polymer}) Merrifield-type macroporous polymer resin (chloromethylpolystyrene with 5.5% divinylbenzene as crosslinking agent) in an anthraquinone-2- carboxylic acid solution (0.467 g) and 0.271 mL of 1,8-diazabicyclo[5.4.0]undec-7-ene (DBU) in 10 mL of *N*-methyl-2-pyrrolidone. The suspension is stirred on an orbital shaker at 150 rpm at 70°C for 24 hours. Subsequently, the resulting resin is filtered, washed with DMF (25 mL x 3), dichloromethane (25 mL x 3) and methanol (25 mL x 3) and vacuum dried overnight at 50°C to obtain 0.6641 g of cat-III for further use in hydrogen peroxide photosynthesis.

### Example 5: The photosynthesis of hydrogen peroxide using immobilised photocatalysts.

Different amounts of immobilised catalyst (listed in Table 5) were suspended in 5 mL of isopropanol. The generation of hydrogen peroxide was produced by exposing the system to 365 nm UV light with continuous stirring and air bubbling for one hour at room temperature.

Table 5 summarises the results obtained for the different catalysts evaluated, as well as the effect of the amount of catalyst used. The H₂O₂ concentration was determined by redox titration with a 0.1 M KMnO₄ aqueous solution. The H₂O₂ concentration obtained was in the range of 1000-3000 ppm, which is in the H₂O₂ concentration range required for peracid synthesis for disinfection and sterilisation applications.

The immobilised catalyst containing anthraquinone-2-carboxylic acid produces a greater amount of hydrogen peroxide (up to 3100 ppm) compared to the one containing anthraquinone-2-sulphonic acid (only up to 1394 ppm).

The photocatalyst without ionic liquid units **(cat-III)** produces a significantly lower amount of hydrogen peroxide 258 ppm H₂O₂ vs. 3128 ppm H₂O₂ obtained for the catalyst with ionic liquid units in its structure (cat-I).

**Table 5. Synthesis of H₂O₂ using photocatalysts immobilised on polymeric supports.**

| **Entry** | **Catalyst** | **Amount of catalyst (g)** | **mol AQN%^{a}** | **[H₂O₂], M** | **[H₂O₂], ppm** |
|---|---|---|---|---|---|
| 1 | **cat-I** | 0.005 | 0.045 | 0.043 | 1462 |
| 2 | | 0.010 | 0.076 | 0.067 | 2278 |
| 3 | | 0.015 | 0.122 | 0.073 | 2482 |
| 4 | | 0.020 | 0.168 | 0.057 | 1938 |
| 5 | | 0.025 | 0.214 | 0.081 | 2754 |
| 6 | | 0.050 | 0.428 | 0.092 | 3128 |
| 7 | **cat-II** | 0.005 | 0.045 | 0.029 | 986 |
| 8 | | 0.010 | 0.076 | 0.036 | 1224 |
| 9 | | 0.015 | 0.122 | 0.029 | 986 |
| 10 | | 0.020 | 0.168 | 0.041 | 1394 |
| 11 | | 0.025 | 0.214 | 0.032 | 1088 |
| 12 | | 0.050 | 0.428 | 0.032 | 1088 |
| 13 | **cat-III** | 0.050 | 0.428 | 0.0076 | 258 |

Reaction conditions: 5 mL of IPA (65.39 mmol), 1 hour, constant stirring, air bubbling, room temperature. a: Percentage with respect to IPA.

## Claims

1. A method for the preparation of an active disinfection and/or sterilisation solution comprising the following stages:
a) a liquid mixture of a primary or secondary alcohol and a dialkyl carbonate is placed in contact with O₂ in the presence of a photocatalyst and UV light to obtain hydrogen peroxide (H₂O₂) by reacting O₂ with the alcohol present in the mixture by means of a photocatalytic reaction,
b) the solution obtained in stage a) is placed in contact with a catalyst for the perhydrolysis reaction of the dialkyl carbonate, this catalyst being non-enzymatic or enzymatic, at a temperature between 25 and 80°C, to form peroxycarboxylic acid (PCA) by reaction of the hydrogen peroxide formed in the previous stage a) and the dialkyl carbonate present in the original mixture,
c) the solution obtained in stage b) is diluted with water to a concentration of said solution in water equal to or less than 10% v/v.

2. The method, according to claim 1, wherein the mixture comprises between 20 and 80% v/v of alcohol and the rest of dialkyl carbonate.

3. The method, according to claim 1 or 2, wherein the alcohol has the following formula: R₁-CH(OH)-R₂, where R₁ is H or a C1-C6 alkyl group and R₂ is a C1-C6 alkyl.

4. The method, according to any of the preceding claims, wherein the alcohol is selected from the list comprising: isopropanol, ethanol, methanol, butanol and glycerol.

5. The method, according to any of the preceding claims, wherein the dialkyl carbonate has the following formula: R₃-O-(C=O)-O-R₃ where R₃ is a linear or branched C1-C6 alkyl, optionally substituted with at least a hydroxyl (-OH) or a C1-C4 alkoxy group or combinations thereof.

6. The method, according to any of the preceding claims 1 to 4, wherein the dialkyl carbonate is cyclic and has the following formula: 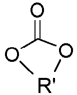 where R' is a C2-C6 alkylene group optionally substituted with at least one hydroxyl (-OH), a C1-C4 alkyl group, a C1-C4 alkoxy or combinations thereof.

7. The method, according to any of the preceding claims, wherein the dialkyl carbonate is selected from the list comprising: dimethyl carbonate, diethyl carbonate, propylene carbonate and glycerol carbonate.

8. The method, according to any of the preceding claims, wherein the photocatalyst used in stage a) is selected from the list comprising: anthraquinone, 2-bromomethylanthraquinone, 1,8-dihydroxyanthraquinone, anthraquinone-2-carboxylic acid, sodium anthraquinone 2-sulphonate, 2-tert-butylanthraquinone, 2-methylanthraquinone, and 2-ethylanthraquinone.

9. The method, according to any of the preceding claims, wherein the photocatalyst in stage a) is in a concentration between 5 and 10 mM.

10. The method, according to any of the preceding claims, wherein stage a) is carried out in a photocatalytic reactor.

11. The method, according to any of the preceding claims, wherein the photocatalyst from stage a) is immobilised in the photocatalytic reactor or else dissolved in the starting mixture of alcohol and dialkyl carbonate.

12. The method, according to any of the preceding claims, wherein the catalyst of stage b) is a lipase enzyme.

13. The method, according to any of the preceding claims 1 to 11, wherein the catalyst of stage b) is a non-enzymatic catalyst.

14. The method, according to any of the preceding claims, wherein the catalyst of stage b) is in a concentration between 0.3 and 0.6 g/mL of the reactant mixture.

15. The method, according to any of the preceding claims, wherein the reaction of stage b) is carried out for a period between 20 and 30 minutes.

16. The method, according to any of the preceding claims, wherein said process is carried out continuously, by continuously supplying a flow of reagents without the need for any separation and/or purification stage.

17. The method, according to any of the preceding claims, wherein the photocatalyst from stage a) is immobilised on a polymeric resin, preferably on a Merrifield resin, which may or may not contain ionic liquids.

18. A system for carrying out the method described in any of the preceding claims 1 to 17, comprising:
• a photocatalytic reactor provided with a light source and an O₂ inlet configured to carry out the reaction of stage a),
• a liquid pump configured to introduce the alcohol/dialkyl carbonate starting mixture, contained in a vessel, into the photocatalytic reactor.
• a reactor connected in series with the photocatalytic reactor configured to receive the solution obtained in stage a) and carry out the reaction in stage b),
• a dilution equipment configured to add water to the solution resulting from the second reactor.

19. The system, according to claim 18, where the photochemical reactor comprises a pressure regulator and/or a gas compressor configured to introduce O₂ under pressure into the reactor.

20. The system, according to claim 18 or 19, comprising flow sensors, located at the inlet and outlet of each reactor, to control the supply of reagents to the system.

21. The system, according to any of claims 18 to 20 comprising a light sensor connected to the UV light source configured to control the light applied to the photocatalytic reactor.

22. An active disinfection/sterilisation solution, obtainable by the method described in any of the preceding claims 1 to 17, **characterised in that** it comprises: a peroxycarboxylic acid, hydrogen peroxide, at least one alcohol, and water.

23. The active disinfection/sterilisation solution according to claim 22, comprising:
- water from 90 to 99.9% v/v and
- a mixture of at least the following three components: peroxycarboxylic acid, hydrogen peroxide and at least one alcohol, wherein said mixture represents from 0.1 to 10% v/v.
